# EUROPEAN PATENT APPLICATION

(11) **EP 3 443 906 A1**
(43) Date of publication of application: **20.02.2019**
(21) Application number: 17782074.3
(22) Date of filing: 18.01.2017
(51) Int. Cl.: A61B 6/03

(54) **METHOD, DEVICE AND COMPUTER PROGRAM FOR AUTOMATIC ESTIMATION OF BONE REGION IN CT IMAGE**

(30) Priority: 13.04.2016 JP 2016080086
(71) Applicant: Nihon Medi-Physics Co., Ltd., Tokyo 136-0075 (JP)
(72) Inventor: HAMADA, Kazuo, Tokyo 136-0075 (JP); DAISAKI, Hiromitsu, Tokyo 136-0075 (JP); NISHIDA, Kazumasa, Tokyo 136-0075 (JP)
(74) Representative: TBK
(86) International application number: PCT/JP2017/001459
(87) International publication number: WO 2017/179255

(57) **Abstract**

An exemplary embodiment provides a method including: creating a histogram of pixel values based on a CT image; determining a soft region peak, which is a peak in a soft tissue region in the histogram; and setting, based on the soft region peak, a threshold representing a lower limit of a bin value in a bone region in the histogram. The method may include automatically removing a bed portion from the CT image.

## Description

### Field

The present application relates to a technique of automatically estimating a bone region in a CT image.

### Background

Computed tomography (CT) is the technology of producing tomographic images of objects through the use of radiation. In general, X-rays are used to take tomographic images. CT is particularly used in the medical field, and is used to create tomographic images of skeletons and organs and create three-dimensional images of these.

Paragraph 0027 in Japanese Patent Application Laid-open No. 2013-088386 indicates that a CT image is binarized to obtain positional information on the femur.

### List of references

### Patent Literature

Patent Literature 1: Japanese Patent Application Laid-open No. 2013-088386

### Summary of the Invention

CT images that have not been subjected to processing other than image reconstruction show bones, internal organs, and fat. To observe a specific bone, for example, it is therefore preferred to extract and display only pixels at which the bone appears. To reduce work burden on operators and prevent individual differences in extracted results, it is preferred to automatically perform the processing for extracting a bone region.

The present invention provides a technique for automatically estimating a bone region in a CT image, and includes: creating a histogram of pixel values based on the CT image; determining a soft region peak, which is a peak in a soft region in the histogram; and setting, based on the soft region peak, a threshold representing a lower limit of a bin value in a bone region in the histogram.

In some embodiments, the creating of a histogram may include: creating a reference image that is a binary image from which a bed portion is removed to leave only a human body portion in the CT image; and creating the histogram by using only pixels where data is present in corresponding pixels in the reference image among pixels in the CT image.

In some embodiments, the threshold may be a bin value that is larger than a bin value corresponding to the soft region peak and has a frequency value accounting for a predetermined proportion of a frequency value of the soft region peak.

An embodiment of the present invention provides a computer program including a program instruction configured to cause a device to implement the features described above when executed by processing means in the device. An embodiment of the present invention provides a method to be implemented by a device when processing means in the device executes a program instruction, the method including the features described above.

An embodiment of the present invention provides a device including processing means and memory means having a program instruction stored thereon, the program instruction being configured to cause the device to implement the features described above when executed by the processing means.

### Brief Description of Drawings

FIG. 1 is a diagram for describing the hardware configuration of a system that can embody the present invention.
FIG. 2 is a diagram for describing an exemplary example of processing for automatically estimating a bone region in a CT image.
FIG. 3 includes diagrams for describing how to create a reference image.
FIG. 4 is a diagram for describing how to create a histogram of a CT image.
FIG. 5 is a diagram for describing processing for setting a threshold of a lower limit of pixel values in the bone region.
FIG. 6 is a diagram illustrating an example of a bone image created by pixels extracted based on the threshold.

### Description of Preferred Examples

Referring to the accompanying drawings, exemplary embodiments of the technical concept disclosed in the present application are described below.

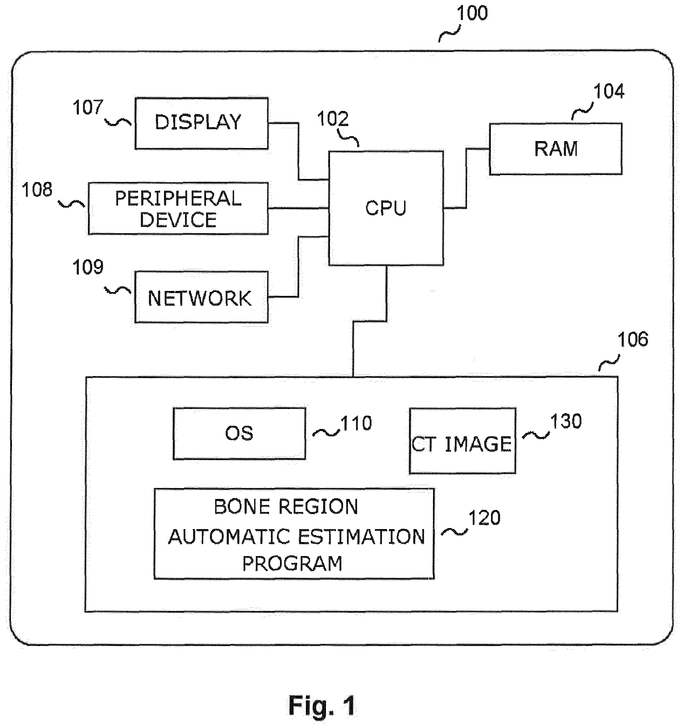

FIG. 1 is a diagram for describing the hardware configuration of a system 100 that can embody the present invention. As illustrated in FIG. 1, the system 100 is similar to a commonly-used computer in terms of hardware, and can include a central processing unit (CPU) 102, a main storage device 104, a mass storage device 106, a display interface 107, a peripheral device interface 108, and a network interface 109. Similarly to a commonly-used computer, a high-speed random access memory (RAM) can be used as the main storage device 104, and an inexpensive large-capacity hard disk or solid state drive (SSD) can be used as the mass storage device 106. A display for displaying information can be connected to the system 100. The display is connected through the display interface 107. A user interface such as a keyboard, a mouse, and a touch panel can be connected to the system 100. The user interface is connected through the peripheral device interface 108. The network interface 109 can be used to connect the system 100 to another computer or the Internet through a network.

The mass storage device 106 stores therein an operating system (OS) 110 and a bone region automatic estimation program 120. The most basic functions of the system 100 are provided by the OS 110 executing the CPU 102. The bone region automatic estimation program 120 includes program instructions related to novel processing disclosed in the present application, and when at least a part of the instructions is executed by the CPU 102, the system 100 can implement the novel processing disclosed in the present application. The mass storage device 106 can also store therein CT images 130. Each of the CT images 130 is three-dimensional image data in which pixel values correspond to respective CT values, and is image data to be analyzed or operated by the program 120.

Other than the components illustrated in FIG. 1, the system 100 may include the same configuration as that of a device included in a general computer system, such as a power source and a cooling device. Various embodiments of a computer system employing various techniques have been known, such as distributed, redundant, or virtualized storage devices, use of a plurality of CPUs, CPU virtualization, use of a processor suitable for a specific process such as DSP, and implementation of a specific process as hardware to be used with a CPU. The invention disclosed herein may be installed on any type of computer systems, and the scope of the invention is not limited by the type of computer systems. The technical concept disclosed in the specification can be generally embodied by (1) a computer program including an instruction for causing, when executed by processing means, a device or a system including the processing means to implement various kinds of processing described in the specification, (2) an operation method for a device or a system implemented by the processing means executing the computer program, and (3) a device or a system including the program and processing means configured to execute the computer program. As described above, a part of software processing may be implemented by hardware.

Note that CT images 130 are not stored in the mass storage device 106 in many cases at the time of manufacture, sales, and initial start-up of the system 100. Each of the CT images 130 may be, for example, data transferred from an external device to the system 100 through the peripheral device interface 108 or the network interface 109. It should be understood that the scope of the invention disclosed herein is not limited whether CT image data is stored in a storage device.

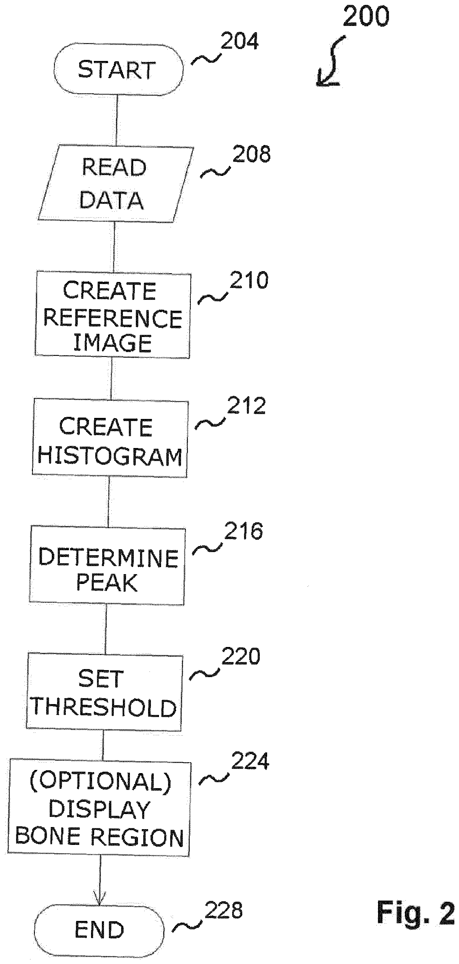

Next, the procedure of automatic estimation processing 200 for a bone region in a CT image disclosed in the present application is described with reference to FIG. 2. The processing 200 can be implemented by the system 100 when the bone region automatic estimation program 120 is executed by the CPU 102.

Step 204 represents the start of processing. In Step 208, data to be processed by the bone region automatic estimation program 120 is read (loaded). Specifically, image data 130 is wholly or partially read from the mass storage device 106 and stored in the main storage device 104. The image data 130 may be directly fetched in the main storage device 104 from an external nuclear medical device through the network interface 109.

In a CT image 130 to be processed by the processing 200 in the present example, pixel values representing water are corrected to be zero in advance. Thus, the CT image 130 has some pixels having negative pixel values.

Next, the processing 200 creates a histogram of pixel values in the CT image 130 (Step 212). In some embodiments, a histogram may be created after a bed portion is removed from the CT image 130. Step 210 represents a step for creating a reference image used to remove the bed portion. Step 210 is performed as follows.

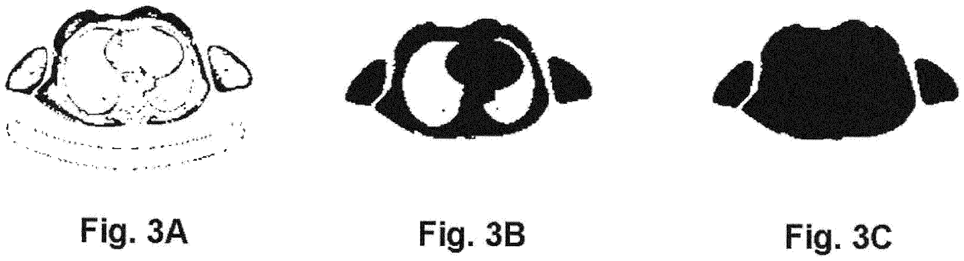

(Step 210A) First, the processing 200 creates a binary image obtained by binarizing the CT image 130. A threshold for the binarization may be a threshold with which a human body is highly likely to be extracted. For example, in the present example, the CT image 130 is corrected in advance such that pixel values representing water are zero as described above, and hence, for example, a binary image may be created by using -190 as a threshold. FIG. 3A illustrates an example of the created binary image.

(Step 210B) Next, the processing 200 searches a vertical slice in the body axis direction from the center of the created binary image for a location with data. Then, region growing processing is performed from the location to create a binary image from which the bed portion has been removed. FIG. 3B illustrates a binary image created by processing the binary image in FIG. 3A in this manner.

When region growing has failed, a binary image is created by removing a bed by 3D labeling processing. The largest label is regarded as a human body, and portions other than the human body are removed to extract only a human body portion.

(Step 210C) After the bed is removed, filling processing to fill hole portions in the body with values is performed, and opening processing of morphology operation is performed to remove a bed that has not be completely removed. FIG. 3C illustrates an image created by processing the binary image in FIG. 3B in this manner. The image obtained by the processing in Step 210C is referred to as "reference image".

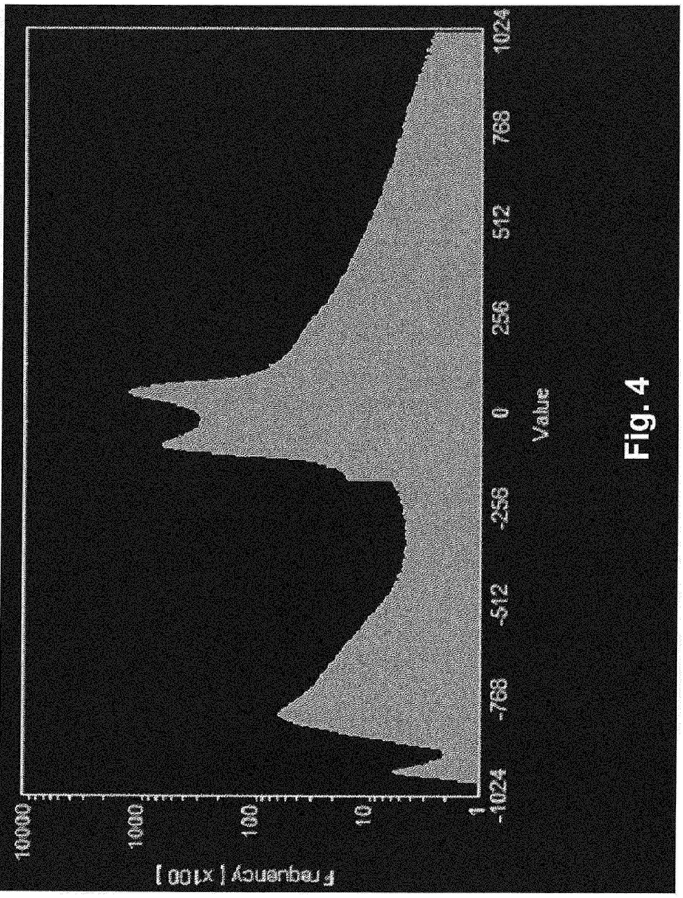

In Step 212, as described above, the processing 200 creates a histogram of pixel values in the CT image 130. In the present example, the histogram is created by using only pixels where data is present in corresponding pixels in the reference image created in Step 210 among pixels in the CT image 130. FIG. 4 illustrates an example of the created histogram. As described above, the CT image 130 is corrected in advance such that pixel values representing water are zero. The CT image 130 is normalized in advance such that pixel values are distributed in the range of -1024 to +1024. Thus, the range of the horizontal axis (bin value) in the exemplified histogram is -1024 to +1024.

In an embodiment in which Step 210 is not performed, a histogram may be created by using the entire CT image 130.

In Step 216, the processing 200 detects peaks of the created histogram. As illustrated in FIG. 4, in a histogram of an X-ray CT image for living subjects, two peaks are generally observed near the center (near a bin value of 0, which is a bin value corresponding to water, in the present example). Data around a peak having a smaller bin value (peak on the left side in the screen) corresponds to a fat region, whereas data around a peak having a larger bin value (peak on the right side in the screen) corresponds to a soft tissue region (organs or muscles). The peak having a larger bin value is herein referred to as "soft region peak". A flat region on the right side of the soft region peak corresponds to a bone.

A peak that is intended to be detected in Step 216 is a soft region peak. Hence, the peak detection processing is not required to be performed in the entire range of bin values, but only needs to be performed in the range of bin values with which it has been experientially known that a soft region peak is present. For example, in a CT image corrected and normalized such that pixel values corresponding to water are 0 and the range of pixel values is -1024 to +1024, the inventors of the present invention have confirmed that a soft region peak of the histogram is distributed in the range of bin values of 0 to 256 in most cases. Thus, the soft region peak detection processing in Step 216 may be performed in a range of bin values of 0 to 256.

In some embodiments, peak detection may be performed in the range of bin values that seem to include a fat region and a soft region. As described above in relation to FIG. 4, two peaks are generally found, and hence one of the peaks having a larger pixel value may be determined to be a soft region peak.

In some embodiments, the peak may be determined by subjecting a raw histogram to smoothing processing.

In Step 220, a threshold of a lower limit of a bin value in the bone region is automatically set based on the soft region peak determined in Step 216. The reason why the threshold is set based on the soft region peak is that pixels in a flat region on the right side of the soft region peak correspond to a bone as described above. Various specific setting processes may be used as long as the processes are based on the soft region peak.

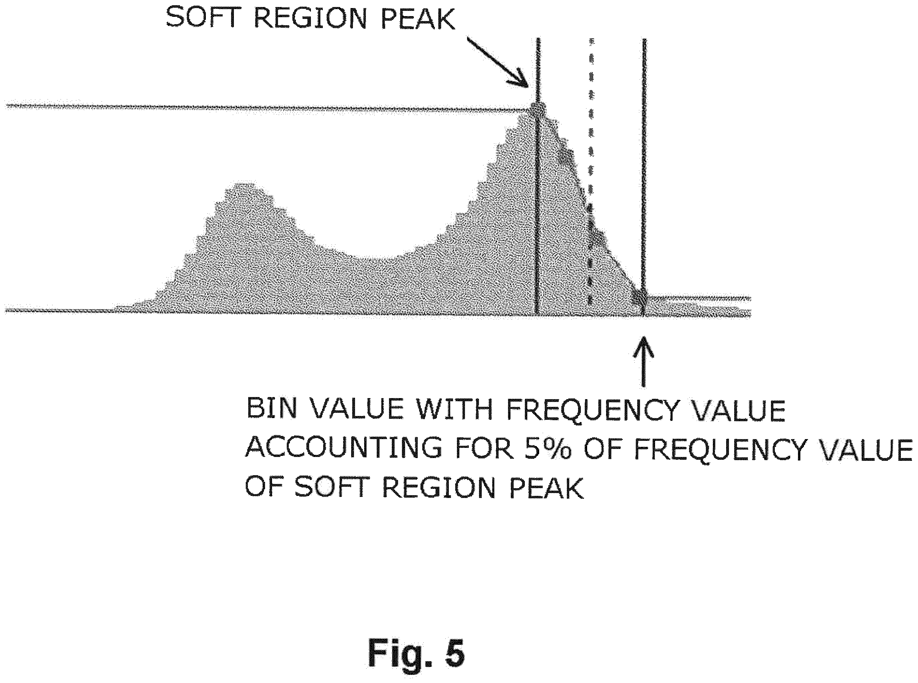

In one example, the threshold may be defined as a bin value that is larger than a bin value corresponding to the soft region peak and has a frequency value accounting for a predetermined proportion of a frequency value of the soft region peak. FIG. 5 illustrates how the threshold is determined in this manner.

As the above-mentioned predetermined proportion, the inventors of the present invention have confirmed that 5%, for example, is one appropriate value. However, the proportion may be another value such as 10%. A proportion that is set in advance based on data on a plurality of subjects may be used. When the above-mentioned predetermined proportion was 5%, a threshold in the histogram illustrated in FIG. 4 was 108.

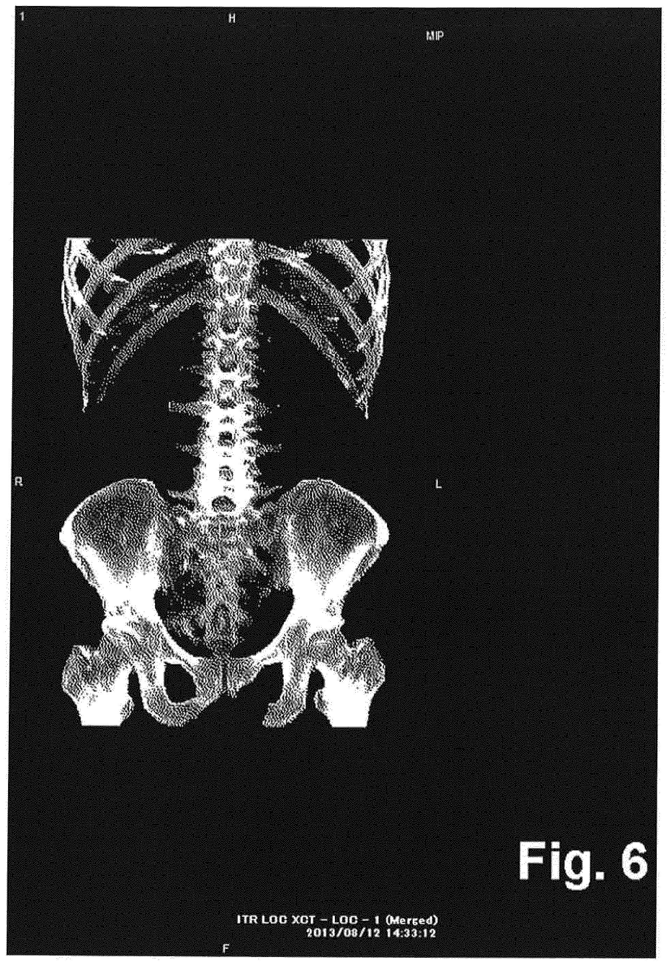

Step 224 is an optional step. At this step, the processing 200 displays the CT image 130 by using only pixels that have pixel values equal to or larger than the threshold set in Step 220 (and pixels where data is present in corresponding pixels in the reference image). Thus, as illustrated in FIG. 6, for example, an image in which only a skeleton appears can be displayed. In this case, as indicated by a histogram under the skeleton image in FIG. 5, an upper limit value of pixel values of pixels representing a bone region may be set as well. For example, the upper limit value may be 500. In other words, the skeleton image in FIG. 6 is an image created by using only pixels having pixel values between 108 to 500 among pixels included in the CT image 130.

Step 228 represents the end of processing.

The processing 200, which automatically estimates the lower limit value of pixel values in a bone region in a CT image, enables the bone region to be automatically extracted and reduces work burden on operators. Because the bone region extraction processing is automatically performed, bone region extraction results can be prevented from varying due to individual differences among operators.

It is preferred that the lower limit value (or upper limit value) of pixel values in a bone region be manually changeable by an operator in order for the operator to flexibly observe a CT image.

While the invention of the present application has been described above in detail by way of preferred examples, the above description and the appended drawings are not presented for the purpose of limiting the scope of the invention of the present application, but are presented in order to meet the legal requirements. The embodiments of the invention of the present application has various variations other than the ones introduced herein. For example, various kinds of numerical values indicated in the specification or the drawings are all illustrative, and these numerical values are not presented for the purpose of limiting the scope of the invention. Individual features included in various kinds of examples introduced in the specification or the drawings are not limited to usage with examples in which these features are explicitly described to be included, but may be used in combination with other examples described herein or various kinds of specific examples that are not described herein. In particular, the processing illustrated in the flowchart is not necessarily required to be executed in the order stated herein. According to the preference of an implementor or if necessary, the processing may be executed in another order or simultaneously in parallel, and a plurality of blocks may be implemented integrally or in a loop as appropriate. These variations are all included in the scope of the invention disclosed herein, and the scope of the invention is not limited by the embodiments of the processing. The described order of the processing defined in the claims is not necessarily required to specify the essential order of the processing. For example, an embodiment specifying a different order of the processing and an embodiment that executes the processing in a loop are also included in the scope of the invention as in the claims.

In addition, for example, embodiments of the bone region automatic estimation program 120 include an embodiment in which the bone region automatic estimation program 120 is a single computer program and an embodiment in which the bone region automatic estimation program 120 is a program group formed by a plurality of independent computer programs. As well known, there are various embodiments of computer programs, and these variations are all included in the scope of the invention disclosed herein.

It should be noted that the applicant claims the right to have a patent granted on all the embodiments not deviating from the spirit of the invention disclosed herein regardless of whether a patent is claimed in the current set of the accompanying claims.

### Reference Signs List

- 100: system
- 102: CPU
- 104: main storage device
- 106: mass storage device
- 107: display interface
- 108: peripheral device interface
- 109: network interface
- 120: bone region automatic estimation program
- 130: CT image data

## Claims

1. A method for automatically estimating a bone region in a computed tomography (CT) image, the method comprising:
creating a histogram of pixel values based on the CT image;
determining a soft region peak, which is a peak in a soft tissue region in the histogram; and
setting, based on the soft region peak, a threshold representing a lower limit of a bin value in a bone region in the histogram.

2. The method according to claim 1, wherein the creating of a histogram comprises:
creating a reference image that is a binary image from which a bed portion is removed to leave only a human body portion in the CT image; and
creating the histogram by using only pixels of the CT image where data is present in corresponding pixels in the reference image.

3. The method according to claim 1 or 2, comprising performing smoothing processing on the histogram before the determining of a soft region peak.

4. The method according to any one of claims 1 to 3, wherein the determining of a soft region peak comprises performing peak detection processing in a predetermined bin value range.

5. The method according to any one of claims 1 to 3, wherein the determining of a soft region peak comprises detecting peaks of the histogram in a bin value range including a fat region and a soft region, and determining one of the detected peaks that has a largest bin value to be the soft region peak.

6. The method according to any one of claims 1 to 5, wherein the threshold is a bin value that is larger than a bin value corresponding to the soft region peak and has a frequency value accounting for a predetermined proportion of a frequency value of the soft region peak.

7. A device comprising:
processing means; and
memory means,
the memory means including a program instruction,
the program instruction being configured to cause the device to implement the method according to any one of claims 1 to 6 when executed by the processing means.

8. A computer program comprising a program instruction configured to cause a device to implement the method according to any one of claims 1 to 6 when executed by means in the device.
